# EUROPEAN PATENT APPLICATION

(11) **EP 3 508 201 A2**
(43) Date of publication of application: **10.07.2019**
(21) Application number: 19153695.2
(22) Date of filing: 08.08.2005
(51) Int. Cl.: A61K 31/198, A61K 9/00, A61K 8/00, A61K 8/44, A61Q 19/00, A61P 17/00, A61P 17/06, A61K 9/20, A61K 9/28, A61K 9/48

(54) **GLYCINE AS A DIET SUPPLEMENT FOR USE IN THE TREATMENT SKIN PROBLEMS THAT RESULT FROM UNDERLYING COLLAGEN DISORDERS AND AND COSMETIC USES THEREOF**

(30) Priority: 09.08.2004 US 59990804 P
(62) Divisional of application: 05850750.0
(71) Applicant: Melendez Hevia, Enrique, 38202 Tenerife (ES)
(72) Inventor: Melendez Hevia, Enrique, 38202 Tenerife (ES)
(74) Representative: Carvajal y Urquijo, Isabel

(57) **Abstract**

A method of treating or preventing a subject's health problem that is related to an underlying disorder in the functioning of the metabolic system of the subject includes the step of administering a therapeutically effective dosage of glycine. For health problems associated with the subject's bones, cartilage or connective tissue, including degenerative diseases such as arthrosis and arthritis, injuries due to physical trauma, the condition of one's skin, the administering step involves daily ingesting glycine at a rate in the range of 0.1 to 0.7 g/Kg of the subject's body weight.

## Description

### CROSS-REFERENCE TO RELATED APPLICATION

This application claims the benefit of U.S. Provisional Patent Application No. 60/599,908, filed August 9, 2004 by Enrique Meléndez Hevia.

### BACKGROUND OF THE INVENTION

### 1. FIELD OF THE INVENTION

This invention relates to new therapeutic uses for glycine.

### 2. DESCRIPTION OF THE RELATED ART

The development of biomedicine in the last fifty years has significantly increased life expectancy, which for a child born today is, in developed countries, around 80 - 90 years, with a mean difference between women and men of about 5-10 years in favor of women. With this increase in the number of elderly has come a significant increase in the reported frequency of degenerative diseases that affect one's connective tissue, cartilage and bone. Most of these diseases are not fatal, but result for those afflicted is a significant decrease of their physical ability due to progressively worsening pain levels.

At the present, the primary cause of such degenerative diseases is unknown. Medical researchers have postulated many possible causes, with many of these speculating about underlying problems in the metabolism of those afflicted. For example, for arthrosis, the most common of these degenerative diseases, such speculations include: a deficiency in proteoglycan biosynthesis or the synthesis of collagen.

Such speculations have led me to refer to these afflictions as "deficiency" rather than "degenerative" diseases. Furthermore, I herein disclose various treatment means for remedying such metabolic deficiencies, with the key to my treatments being based on the hypothesis that dietary changes can influence designated metabolic processes so as to rectify any deficiencies that they may have. Numerous clinical results are presented herein which attest to the effectiveness of my treatment methods. These methods generally involve the daily consumption of relatively large amounts of glycine.

Glycine has gained some popularity in recent years in the ever-expanding "diet supplement" industry as an orally administered substance which its manufacturers claim, advertise and promote as being helpful in addressing an assortment of health issues. However, it should be noted that none of these claims, like those for many other substances in the "diet supplement" industry, are as yet supported by extensive medical research or clinical trials. The claims include, for glycine consumption rates generally recommended in the range of 0.5 to 1.0 grams/day, the benefits of: (a) promotion of deeper sleep, (b) adult memory retention, (c) supporting healthy functioning of the kidneys, liver and nervous system, and (d) development of a strong immune system,

The patent literature does disclose various medical-related uses for glycine. For example, U.S. Patent No. (USPN) 5,854,286 discloses the use of orally administered glycine in dietary quantities (i.e., 35 grams/day or 0.4 g/(Kg of body weight)/day) for the treatment of schizophrenia.

USPN 6,025,327 discloses the oral consumption of low doses of hydrolyzed collagen, or the products of its hydrolysis, or even a selected mixture of amino acids (but without a specific mention of glycine as a necessary ingredient) for the treatment of degenerative joint diseases. The recommended dosages of up to 4 g/day of hydrolyzed collagen type II (with ∼ 9 % glycine) equates to about 0.36g/day of glycine.

USPN 6,048,543 discloses a method for treating a human having elevated tumor necrosis factor levels by the administration of an effective amount of one of the amino acids: glycine, alanine and serine. They describe three mixtures with a broad variety of compounds, glycine among them, but at an amount equivalent to other amino acids or other products, and always far from any dosage in the range of 10g/day.

USPN 6,281,244 discloses the daily intake of glycine to prevent or treat acute or chronic graft rejection, optionally in combination with an immune suppressant or an immune modulating agent. This disclosure makes no mention of the applicability of such methods for treating arthrosis or related diseases.

USPN 6,310,097 discloses a method for preventive and/or the therapeutic treatment of cerebral dysfunction. This method involves administering a preventively and/or therapeutically effective amount of a composition comprising at least one of L-serine, glycine, fatty acid compounds thereof, physiologically acceptable salts thereof, hydrates thereof, and solvates thereof.

USPN 6,331,569 discloses a method for improving hair growth, skin structure and/or nail regeneration. The method involves administering a preparation comprising a mixture of amino acids (up to 10 wt. % glycine) and an effective amount of proline and at least one carrier. It is further stated that the preparation may have a positive effect on a number of other functions, including muscle build up, and the firmness of the connective tissue.

USPN 6,100,287 discloses a mixture of amino acids, essentially L-arginine and glycine, and other mixtures with different amino acids for enhancing muscle performance and recovery from fatigue. The practice of sports people consuming different mixtures of amino acids is well known in the art.

Although this listing of patents might appear to contain a large number of uses for glycine, my research has revealed that there are many more health conditions that can be aided by having those afflicted to orally consume relatively large daily amounts of glycine. Additionally, the effective consumption amounts that I have discovered are far outside the range of those generally being advocated (i.e., < 1g/day) in the previously disclosed usages for glycine.

Thus, despite the prior art, the opportunity still appears to exist for one to identify how an existing dietary supplement (i.e., glycine) can be used more effectively to treat various health conditions that have not previously been recognized as benefiting from such new treatment methods.

### 3. OBJECTS AND ADVANTAGES

There has been summarized above, rather broadly, the prior art that is related to the present invention in order that the context of the present invention may be better understood and appreciated. In this regard, it is instructive to also consider the objects and advantages of the present invention.

It is an object of the present invention to provide a diet supplement for the treatment of a wide range of health problems that result from underlying disorders in the functioning of one's metabolic system.

It is also an object of the present invention to provide an improved treatment for degenerative diseases affecting connective tissue, cartilage and bone.

It is an object of the present invention to provide a treatment for health problems that arise because of the body's inability to build adequate number of red blood cells.

It is another object of the present invention to identify a food supplement that can be taken on a regular basis to treat for degenerative diseases affecting connective tissue, cartilage and bone.

It is yet another object of the present invention to provide a food supplement that can be taken on a regular basis to treat a wide range of human and animal physical conditions in which the affected parts of the body are its connective tissues, cartilage and bones, or in which a health problem has arisen because of the body's inability to build adequate number of red blood cells.

It is a further object of the present invention to identify a food supplement that humans can be taken on a regular basis to treat a wide range of health conditions in which the affected parts of the body are its connective tissues, cartilage and bones and the affected bodily processes include the building of red blood cells. These health conditions include some that are relatively obvious, such as: degenerative diseases such as arthrosis and arthritis, injuries due to physical trauma, multiple sclerosis, assorted muscular problems including muscular dystrophy, birth defects such as scoliosis, osteoporosis, anemia and others which are probably nonobvious, such as: susceptibility to infectious diseases, asthma, elevated cholesterol levels, cancer, infant strength levels, and assorted health problems with one's skin, teeth and gums, finger nails, eyes, ears and vocal chords.

It is a still further object of the present invention to identify a food supplement that can be administered to animals to treat them for a similar wide range of health conditions in which the affected parts of animal's body are its connective tissues, cartilage and bones.

It is also an object of the present invention to provide a dietary supplement that will decrease the extent of heath problems in which the affected body parts are its connective tissues, cartilages and bones.

It is a further object of the present invention to provide a cream can be applied directly on one's skin to address a number of skin problems, including the treatment and prevention of skin lines, wrinkles, and other more severe skin problems, such as psoriasis.

These and other objects and advantages of the present invention will become readily apparent as the invention is better understood by reference to the accompanying summary, drawings and the detailed description that follows.

### SUMMARY OF THE INVENTION

Recognizing the opportunity the identification of additional uses for glycine, the present invention is generally directed to exploiting this opportunity and thereby contributing new treatment method for a wide range of health problems.

In a first embodiment, the present invention is a method of treating or preventing a subject's health problem that is related to an underlying disorder in the functioning of the metabolic system of the subject includes the step of administering a therapeutically effective dosage of glycine. For health problems associated with the subject's bones, cartilage or connective tissue, including degenerative diseases such as arthrosis and arthritis, injuries due to physical trauma, the condition of one's skin, teeth, gums, finger nails, eyes, ears, vocal chords or muscles, or diseases such as osteoporosis, asthma, multiple sclerosis or atherosclerosis, the administering step involves daily introducing into the subject's body glycine at a rate in the range of 0.1 to 0.7 g/Kg of the subject's body weight.

In a second embodiment, the present invention is a compound containing the appropriate amount of glycine that allows the compound to be effectively used as part of the process of performing the method which is the first embodiment of the present invention.

In a third embodiment, the present invention is a method for improving an aspect of the well being of a subject, wherein the aspect is related to the functioning of the metabolic system of the subject, and the method includes the step of administering a therapeutically effective dosage of glycine. Examples of some of these aspects include: the ability of the subject to recover from a surgical procedure, the subject's growth rate or strength levels, the ability of the subject to forestall the contraction of an infectious disease, and the athletic performance levels of the subject.

Thus, there has been summarized above, rather broadly, the present invention in order that the detailed description that follows may be better understood and appreciated. There are, of course, additional features of the invention that will be described hereinafter and which will form the subject matter of the claims to this invention.

### BRIEF DESCRIPTION OF THE DRAWINGS

FIG. 1 shows the age distribution of the study participants for the (a) total population (■---■), (b) population of those afflicted with arthrosis (Δ-Δ), and (c) population of those afflicted with physical injuries (○ - ○).

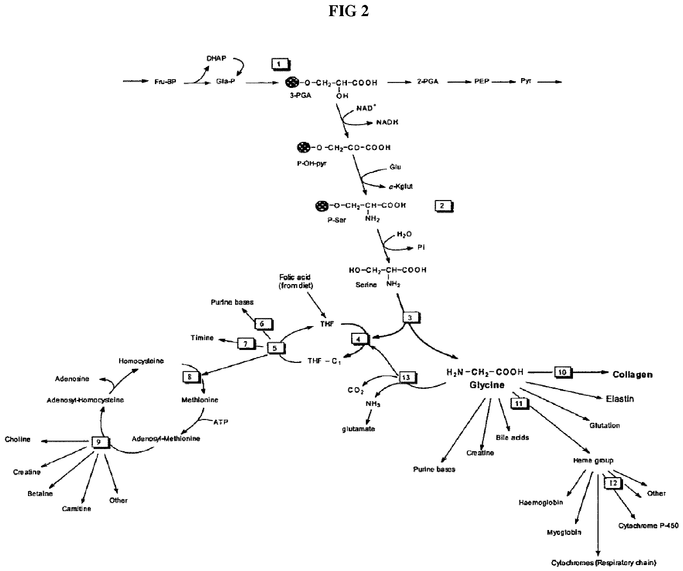

FIG. 2 shows the metabolic pathways involved in glycine biosynthesis and its use for different metabolic ends. (1) glycosis, the central pathway of carbohydrate metabolism; (2) specific branch which starts from glycolysis to yiels serine and glycine; (3) Glycine hydroxymetiltransferase EC 2.1.2.1, reaction that converts serine into glycine with the participation of tetrahydrofolic acid (THF) to take the Ci unit; (4, 5 and 6) set of reactions that lead to other processes, necessary in glycine biosynthesis to unload the C₁ unit from THF, and so to have THF ready to participate in Reaction (3) again; (7) set of biosysthetic processes where glycine is the basic raw material; (8) metabolic reaction leading to transfer the methyl group to adenosyl-methionine; (9) methyl transfer reactions; (10) glycine cleavage system (redox enzyme) EC 1.4.4.2/2.1.2.10.
FIG. 3 shows the structures of heme (the prosthetic group of hemoglobin) and porphobilinogen (the basic intermediate in heme building).
FIG. 4 illustrates the chemical structure of cholesterol, and the bile salt glycocholate, which is synthesized in liver from cholesterol, in a chain of reactions that include the addition of glycine.

### DESCRIPTION OF THE PREFERRED EMBODIMENT

Before explaining at least one embodiment of the present invention in detail, it is to be understood that the invention is not limited in its application to the details set forth in the following description or illustrated in the drawings. The invention is capable of other embodiments and of being practiced and carried out in various ways. Also, it is to be understood that the phraseology and terminology employed herein are for the purpose of description and should not be regarded as limiting.

I believe that I have discovered a very simple means to treat a wide range of human and animal physical conditions in which: (a) these conditions result from underlying disorders in the functioning of one's metabolic system, or (b) the afflicted parts of the body are its connective tissues, cartilage and bones or in which a health problem has arisen because of the body's inability to build adequate number of red blood cells.

To test the effectiveness of the proposed treatments on humans, an experimental group of 600 people (347 females, 253 males) between the ages of 4 and 85 was assembled. They were afflicted by a wide range of physical aliments and injuries or had other medical conditions. Table 1 below shows the diseases and other health problems that were afflicting the study participants.

**Table 1. List of degenerative diseases and other health problems in the experimental group of 600 patients participating in the study.**

| Disease/Health Problem | Number of cases |
|---|---|
| Arthrosis and arthritis | 378 |
| Physical injuries | 320 |
| Skin Problems | 150 |
| Teeth and gum | 146 |
| Finger nail problems | 137 |
| Anemia | 96 |
| Osteoporosis | 65 |
| Disc Hermia | 63 |
| Cholesterol excess | 54 |
| Vision, Presbyopia, cataracts | 46 |
| Asthma | 31 |
| Vocal cords, Impaired voice | 27 |
| Muscular problems | 23 |
| Hearing problems | 20 |
| Cancer | 16 |
| Birth physical defects | 12 |
| Multiple sclerosis | 18 |
| Fibromyalgia | 19 |
| Infertility | 12 |
| Total problems | 1,633 |

Since many patients had multiple health problems, the total number of afflictions (1,633) was higher than the number of study participants (some problems with few documented cases, such as vascular problems, are not included in the list).

These health conditions affecting body's connective tissues, cartilage and bones included some that were relatively obvious, such as: degenerative diseases such as arthrosis and arthritis, injuries due to physical trauma, multiple sclerosis, assorted muscular problems including muscular dystrophy, birth defects such as scoliosis, osteoporosis, and others which are probably nonobvious, such as: susceptibility to infectious diseases, asthma, elevated cholesterol levels, cancer, infant strength levels, and assorted health problems with one's skin, teeth and gums, finger nails, eyes, ears and vocal chords.

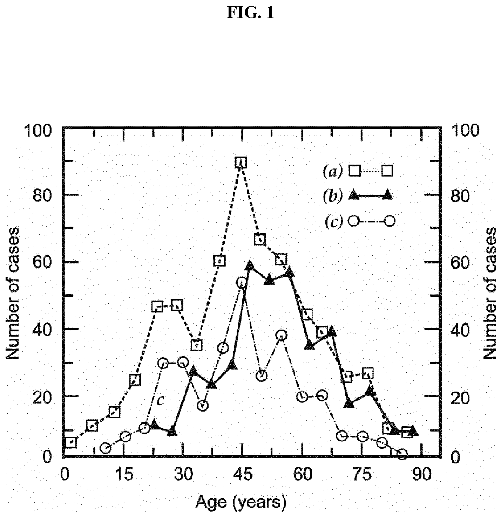

FIG. 1 shows the age distribution of the study participants for the (a) total population, (b) population of those afflicted with arthrosis and (c) population of those afflicted with physical trauma injuries. As evident from the figure, for arthrosis patients, the treatment with glycine was successful in all cases, both in women and men of all ages, with improvements being faster in people under 55 years of age, and even faster in people under the age of 45 years. Similar results were obtained in promoting faster healing of those having physical injuries.

All of the study participants volunteered to participate in this research and they agreed to periodically report the results and any changes that they felt in their symptoms. The also agreed to, in many cases, abandon any other treatment method s that they were formerly using, including analgesics and anti-inflammatory drugs.

The basis of my treatment was to provide study participants a daily dosage of glycine, which is a material authorized in most countries for human consumption as a food additive. The glycine used in this study was purchased from several chemical companies at a quality degree level of nutritional additive.

After some initial trials, it was determined that the regular, daily consumption for study participants would be set at 10 grams (g) of glycine (i.e., approximately 0.1-0.2 g/Kg of a participant's body weight, with a recommended dosage of approximately 0.15 g/Kg for those weighing 5-80 Kg), with this being consumed in doses of 5 grams (equivalent to about one large, coffee spoon) twice per day - in the morning after breakfast, and in the evening, after dinner. Dosages of greater than 5, 10 or 15 grams per day should also be effective and are considered to come within the scope of the present disclosure. It was often dissolved in milk, yogurt, or assorted juices (e.g., orange) and other liquids. It proved to be easily soluble and seemed to add a slight sweet taste to the liquid.

The treatment results observed in this study group for their various health problems, and a few distinct medical conditions, are briefly described below, along with some of my observations on how to extend these results so that they are applicable to animals. My postulated theoretical basis for these results follows thereafter:

Arthrosis and arthritis: Many of the participants had reported having severe pains for years and were using analgesic/anti-inflammatory treatments. They were asked to discontinue these treatments during this study, but were told not to endure any additional pain and to resume taking their usual medication as soon as they need it.

The glycine treatment proved to be effective in all cases; usually in a time period between two weeks and four months and generally according to the participant's age and the nature of the afflicted joint/s. Most of the people under 40 years of age achieved some pain relief in the first week of treatment, while some people over 60 years old needed more than four months to see the first results. Shoulder joints took the longest in which to see improvements.

Many persons with Heberden's or Bouchard's nodes recovered the normal shape of their fingers, although more than one year of treatment was necessary to achieve this result. The participants' conditions steadily improved with time of treatment and many participants eventually reported a full recovery and total lost of pain in all their damaged joints without taking any analgesic or anti-inflammatory drug.

Several additional experiments were conducted with the group of 120 patients specifically affected with arthrosis: (a) the interruption of the treatment of 50 of them (aged between 50-65 years) provoked the reappearance of the pain in their finger joints after some weeks, and, with their return to my glycine treatments, their pain was again eliminated, usually within a few weeks of treatment resumption, (b) the reduction of their dosage to half (4-5 g/day), after several months of treatment, provoked a moderate return to the pain in their fingers, but their return to the initial dose (10 g/day) again produced the same improvements as before.

These results appear to suggest that if one continues with my glycine treatments that they may effectively rid themselves of the symptoms caused by these diseases. My glycine treatments are more that an analgesic, whose only effect is to remove the pain, since for many participants they also greatly reduce the disfigurations associated with prior joint damage. Similarly to how "scurvy is a disease that is said to be cured by taking ascorbic acid," it may be appropriate to say that these degenerative diseases may be "cured" by my glycine treatments.

No negative results (e.g., gastric or intestinal problems, symptoms similar to seasickness that have been reported with other prolonged treatments) were observed with these treatments.

These results suggest that glycine may be considered as an essential nutrient as the body whose required amounts cannot be met by one's usual diet. Therefore, it has to be added daily to one's diet to avoid these degenerative diseases.

Physical Injuries: The persons participating in the study who suffered from recurrent pains as a result of injuries due to physical traumas included amateur and professional sportsmen and others who had injured themselves several years before and had undergone one or more surgeries to correct their continuing problems. Some of them had been using glucosamine treatments without any appreciable improvements.

For a wide range of injuries and consequent bone or joint disorders (e.g., broken bones, tennis elbows, knee or ankle sprains, assorted knee problems), the study participants reported rapid reductions in their pain levels or its frequency of occurrence upon beginning my glycine treatments (daily consumption of 10 grams of glycine). Improvements were reported after only a single week of treatments. In general, those participants whose health problems were caused by traumatic injury responded considerably faster to my glycine treatments than those who problems were due to degenerative diseases. In some instances, daily dosages of 15-20 grams were found to yield even better treatment results.

An interesting group of participants proved to be several young people (ages 17-27) suffering from sports injuries (e.g., ankle sprains and knee injuries), after only one to two weeks of taking my recommended glycine supplements, their joints were sufficiently healed to allow many of them to resume playing their sports.

Skin Problems: After a few weeks of treatment, many participants reported spontaneously, without prior discussion of their conditions or skin concerns, results such as: (a) improvements in their skin's smoothness and shine, including a "chaps" condition, (b) improvement in skin pigmentation conditions, including lentigo and (c) reductions in skin wrinkles and sagging. These effects were so significant as to, in some instances, impact one's general body shape, as in the situation where a women's breasts were observed to be firmer and without as much sag after adhering to recommended daily dosages of glycine. These results are not totally surprising when it is considered that collagen is a major component (around 75%) of the skin. The improvements in collagen biosynthesis as a result of glycine diet supplements would be expected to have an impact on the health of one's skin.

The application of glycine treatments for skin problems and the ease with which creams may serve as a medium by which medications may be applied to skin problems suggests the possibility of developing a glycine-containing cream, lotion, balm, pomade, etc. for the treatment and prevention of skin lines, wrinkles, and other more severe skin problems, including psoriasis. The existence of several commercial skin creams that contain small amount of collagen (e.g., 1 mg of collagen per 100 mg of cream) in their compositions possibly lends further support for the development of a glycine-containing cream.

To test the feasibility of a glycine-containing cream, I prepared creams containing between 500 and 1700 mg of glycine per 100 g of cream. The most concentrated cream was prepared with 800 grams of a commercial neutral base of standard moisturizing cream or body milk, to which was added 100 mL of a distilled water, 15% solution of glycine. These two ingredients were mixed slowly and the mixture was heated to around 40°C, and so this gives a cream with about 1.6% glycine, which is a much higher concentration of the active ingredient than the previously mentioned collagen-containing commercial creams. It should be noted that higher concentrations of glycine appeared to yield a stinging sensation on the skin. For sensitive skins, concentrations of 0.3-0.7% glycine may be advisable. Twice daily applications of these creams were found to yield results that were similar to those previously mentioned for the daily 10 grams, oral dosages of glycine.

Teeth and gum: This study included 146 cases of teeth and gum problems (e.g., tooth pain, gums that bleed easily, a high tendency to have tooth decay). The time of treatment necessary to achieve improved results for such problems was observed to be related to the seriousness of the problem. In some cases, improvements were obtained after only two weeks of treatment, while more severe problems sometimes required many months of treatment (e.g., men in their 50s who had suffered all their lives with bleeding gums when brushing their teeth brushing: total elimination of symptoms within 6-12 months). These results suggest my glycine treatments have slower recuperative periods in some parts of the body than in others. About 30 participants had some type of orthodontic problems and they were also observed to be improved with my treatments. The most interesting and definitive of these cases was a 12-year-old girl suffering from tooth irregularities (i.e., her high jaw was closed and deviated to the right, her palate was very narrow, and her lower jaw bone was short and deviated to the left, and her incisors inclined). Her dentist has established a 2-4 year program of orthodontics, using a succession of bracing devices. Two months after beginning her bracing program, the girl started with my glycine treatments. After another three months, her teeth and the jaw structure were normal, and two months later her dentist removed all her bracing devices. She declared that she had never seen a patient pass from Phase 3 to Phase 1 of an orthodontics program in just five months.

The nature of these health conditions suggests and one's typical oral hygiene habits suggests that other methods could be used to provide the recommended daily doses of glycine. For example, glycine could be added to: (a) toothpastes, (b) mouth washes, (c) oral rinses and (d) pastes, creams, ointments, oils, liquids or patches, etc. used by dentists in their periodontal practices to prevent and fight weak teeth, bleeding gums, and other gum diseases caused by mechanical weaknesses in the afflicted areas.

Finger Nails: This study's 137 participants afflicted with cases of weak or fragile finger nails reported that their conditions were greatly improved after they began taking my glycine treatments. This suggests to me that the origin of many finger nail problems may be a skin basement weakness. It also suggests to me that it may be able for one to treat various hair growth problems with some variation or form of my glycine treatment methods.

Anemia: Anemia is the general name of a broad group of diseases characterized by the occurrence of some problem in the function of red blood cells to take, transport, or delivery oxygen, from the lungs to the tissues. Anemia can have many different origins, as in principle, any failure in the process of red blood cell building, or working, can lead to some problem in oxygen transport, but it is obvious that some causes can be more probable than others.

This study's participants included 96 who suffered from some kind of anemia, with ferropenic anemia (iron deficiency) being the most frequent. My glycine treatment was observed to be effective in all these cases. It suggests to me that some kinds of anemia which are now thought to be due to iron deficiency could more accurately be identified as being due to a glycine deficiency.

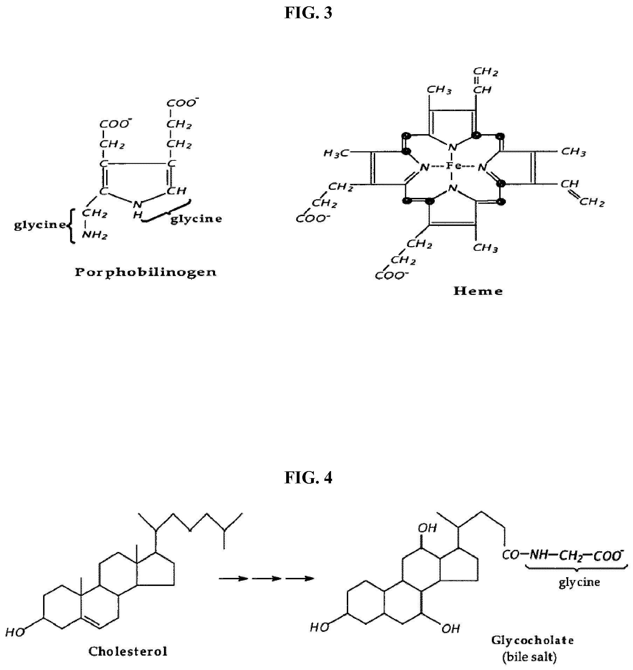

To explain these treatment benefits, it is my observation that there is a closer relationship between anemia and glycine than has heretofore been recognized. FIG. 3, which is discussed in more detail later in this disclosure, shows the structures of heme (the prosthetic group of hemoglobin) and porphobilinogen (the basic intermediate in heme building). It can be seen that eight molecules of glycine are spent to build one heme. Hemoglobin is a very abundant protein in the body representing 90% of the dried weight of the red blood cells (around 2×10¹² cells in the body of an average adult). Moreover, hemoglobin turnover is short (120 days) and there is no recycling of material, as the heme group is destroyed to bile pigments. Blood building is, thus, a high expenditure of glycine, and so its deficiency can be a very probable cause of anemia.

Osteoporosis: The 65 female participants in this study who were afflicted with this disease or condition were all diagnosed, using bone densitometries, as having bone mass losses of between 5-20 %. After several months of my glycine treatments (daily dosage of 10 grams), all of these participants were measured to have reduced the degree of their bone mass losses. These results may eventually demonstrate that the main cause of osteoporosis, initially thought due to calcium deficiency, is actually glycine deficiency, which it is theorized makes the synthesis of the organic support for calcium (collagen) difficult.

Elevated Cholesterol Levels: Hypercholesterolemia or "high blood cholesterol" is the presence of high levels of cholesterol in the blood. It is not a disease but a metabolic derangement that can be secondary to many diseases and can contribute to many forms of disease, most notably cardiovascular disease. 54 people in my experimental group had high blood cholesterol levels, some of them having levels much higher than 280 mg/100 mL. Since many of these participants continued on their prior treatment programs, the results achieved by this study are not somewhat unclear. Only 24 cases of this group were treated exclusively with my glycine treatments, and their cholesterol levels were all decreased so that they fell in the range of 150-180 mg/100 mL.

Vision Problems: All of the 46 participants in this group reported some vision improvements after adhering to my glycine treatment programs (daily dosage of 10 grams). For example, all those with presbyopia reported some vision improvements (i.e., 0.5 or 1.0 diopter after one year of treatment). It is speculated that a daily dosage of glycine to those who are considered too young to yet have such vision problems can actually forestall or postpone the occurrence of such vision conditions.

Asthma, A Chronic Respiratory Disease: Glycine treatments (5-10 grams/day) were observed to effective at reducing or eliminating the participants' asthma problems. Several cases of asthma were in 10-15 year-olds who suffered continuous asthma attacks with frequent crises of noisy breathing, etc. They had to be frequently under corticosteroid treatment. After 30-60 days of glycine treatment, these asthma crises were much less frequent, and when necessary, the corticosteroid treatments lasted just one day. After five months of glycine treatment, the occurrence of asthma crises ceased.

Voice Problems: Study participants who suffered from impaired and fatigued voices reported significant improvements after adhering to my glycine treatments. The mean duration to achieve these improvements was between 2-4 months, with this period being less for younger people. It would appear that glycine intake should be a fixture in the diets of those people (e.g., teachers, singers) whose work significantly depends on the use of their voices.

Muscular Problems: Study participants were afflicted with several different types of problems, including cases of muscular dystrophies. All of these participants reported these conditions to have improved after they began adhering to the recommended glycine treatments (5-10 grams/day).

Hearing Problems: Twenty study participants had hearing problems, some of them very severe. Glycine treatment for them yielded improvements in most of them, including a case with the recovery of audition in one ear. It is my speculation that this result reflects the fact that hearing problems associated with mechanical problems can be solved or improved by glycine treatments.

Cancer: Although this study had few participants who were suffering from cancer, those who added glycine treatments to their other treatments did report some improvements in their conditions. For example, those undergoing chemotherapy treatments and who usually felt quite ill for several days after each session, reported that they tolerated the chemotherapy when they were also adhering to a glycine treatment (i.e., they felt like resuming normal activities just a few hours after each session).

In addition, two cases yielded very good results: (1) A 66-year-old woman with an adenocarcinoma of the colon with numerous metastases. After 20 sessions of chemotherapy, her carcinoembryonic antigen (CEA) was still very high (i.e., 3319 ng/mL, when the normal value for a healthy person is <5.0 ng/mL) and her other test results were also consistent with an advanced cancer (i.e., LDH = 821, GGT = 125, and alkaline phosphatase = 128). She began my glycine treatment while continuing her chemotherapy (i.e., oxaliplatino and continuous infusion of 5-fluorouracile) and twenty-five days later her CEA value lowered to 154 her LDH returned to its normal value, with these improved values continuing to be observed over the following months that she was observed; (2) A 55 year-old woman who had a metastatic mammary carcinoma with bone metastasis and poor treatment prospects began glycine treatments and within two months was in absolute tumoral remission (note: this was checked and demonstrated by three ways: (a) computerized axial tomography (TAC) -total body high resolution, (b) positron emission tomography (PET), and (c) bone gammagraphy).

I am speculating that these results are achieved because of the fact that daily or periodic glycine intake helps the body to strengthen and reinforce its connective tissues which impedes the advance of invasive agents, even cancer cells, bacteria or viruses. My recommended daily glycine intakes may eventually prove to be a vital means to impede tumor growth.

Birth Defects: This study group consisted of twelve participants who had physical malformations or deformities in the mechanical structure of their bodies. Under glycine treatment, all of them reported improvements. Perhaps the most spectacular case was a 5-year-old boy who had a strong progressive scoliosis that was detected a few weeks after his birth. At the age of two years, he was provided with a Milwaukee Brace. In spite of it, at the age of five he had a spinal deviation of 55 degrees to the left and a prognosis of a worsening condition. However, after three months of treatment with a regular intake of 10 grams glycine daily, his spinal deviation had been reduced to 39 degrees, and he had grown 5 centimeters of height.

Multiple Sclerosis: This study's 18 participants all reported improvements in their conditions upon adhering to my glycine treatments. The improvements included noticeable strength increases, and the ability to perform physical exercises that were not able to perform prior to my glycine treatments.

Effects On The Health of Babies Whose Mothers Intake Glycine Daily: The impact on the health of babies whose mothers intake glycine on a daily basis has been investigated in only a few cases. My initial, preliminary results are as follows: Mothers who were under glycine treatment for two years, remained under glycine treatment (some of them increased the daily intake to 15-20 g) during all of their pregnancy. Their babies were born perfectly and noted as being especially strong. This feature was particularly noticed in the infants' necks; it is well known that new-born babies have great difficulties in raising or turning their head, which can place them at a risk of drowning themselves if they are allowed to sleep face up until they their necks develop enough strength to move their heads. However, it was surprisingly observed that the babies born to glycine consuming mothers were able to raise and to turn heads with days of their birth. Additionally, they showed other demonstrations of strength in hands and legs. These numbers of test cases on this matter are not yet sufficient to warrant strong conclusions being drawn from these initial results, but they are consistent with my predictions for such benefits.

Impact of Glycine Intake On One's Susceptibility to Infectious Diseases: Many persons in this study reported that, after starting the glycine treatment, they had less infectious diseases (e.g., sore throat, flu, or cold) as compared to number of instances of such problems that they usually had in prior years. Since there was no control on these participants for such matters, it is difficult to draw any conclusions from these observations. The spontaneous declaration of such observations was unexpected, but possibly not unpredictable. As previously mentioned, I have postulated that glycine intake increases the reinforcement potential of the body's connective tissues, which may conceivably impede the advance of invasive agents (viruses or bacteria), thereby enhancing the capability of the immune system to repel such invasive agents in the same way as it is postulated that glycine strengthened tissues may repel cancer cells.

Extension of My Experimental Results To Domestic Animal Health Problems: The natural food of domestic dogs (just as for wolves and all other mammalian *Canidae)* is made up basically by bone, cartilage, and the skin of their preys (i.e., a set of materials rich in collagen and thus glycine). However, at the present, this food has been replaced by an artificial combination of different products that, I believe, do not have enough collagen and glycine. Furthermore, I believe that the current situation, in which virtually the entire domestic dog population is afflicted with arthrosis, is a consequence of this food switch and its resulting glycine deficiencies in our dogs' diets.

Let us make some calculations. The natural food of wild dogs, that weigh 30-40 kg, is about 1 kg of meat (whole meat, including the material mentioned above) per day. Let us suppose that the parts of this meat that is rich in collagen is about half of it. Assuming that glycine is 20% of the mass in collagen, and 5% in other proteins, and that proteins are 20% of the animal tissues, it gives: 1 kg of meat contains 200 g of proteins, of which 25 g is glycine. This means that 25 g of glycine is the mean daily diet intake of a wild 30-40 kg dog. Meanwhile, artificial dog food that is made mainly of carbohydrates and the intake of glycine for such a dog eating such food is only about 1-2 g/day.

These calculations suggest that the taming of dogs may have made them susceptible to degenerative diseases. This situation seems not to be so dramatic in cats, probably because they are much more carnivorous, and many of them do not accept a highly-carbohydrate content food.

These calculations suggest that we should complement our dogs' diets with daily glycine intakes (0.1 - 0.3 g of glycine per every Kg of body weight for adult animals, and 0.3 - 0.5 g per every Kg of body weight for puppies and other young animals).

Meanwhile, horses, donkeys and other domestic non-ruminant herbivorous almost surely also have a problem with inadequate daily glycine intakes that can promote their premature aging, as manifested by fatigue, loss of strength and their clumsy movements. Cows and bulls, even though ruminants, may also have glycine needs because once they are domestic animals they no longer have natural food selections which can supply their glycine needs. They thus are more prone to bone and joint injuries.

It is my belief that these potential problems can be remedied by adding appropriate amounts of glycine to the food of such animals. Their dosage should probably be about the same as that for domesticated dogs.

Some experimental verification for these beliefs is given by the fact that four dogs afflicted with some kind of arthrosis or arthritis were treated with the same regular dosage as recommended for humans (1 g daily for each 5-7 Kg of body mass), The results in their improvement movement and vitality were apparent two weeks after starting the treatment, and their ambulatory problems disappeared totally over the next two months and enabled them to be removed from their prior anti- inflammatory treatments.

I anticipate that many who learn of these results will initially think that they are too grand and significant for such a simple treatment method. However, I believe that they may be achievable for the following reason, which will be explained below: the body's cells create a large demand for glycine which our metabolic systems do a very poor job of meeting. This realization can possibly help to explain the origins of many degenerative diseases.

The glycine treatment method that I have discovered is a means for helping the body to meet its heretofore unrecognized glycine demand. I have found glycine to be an absolutely necessary nutriment that is indispensable for a correct working of one's metabolism. I believe that the metabolic use of glycine is structural, not regulatory, and not catalytic; thus, it is necessary that it always be available in large amounts in our bodies.

I believe that my treatment methods are feasible since glycine can be considered a natural nutriment (i.e., it is not a drug). Thus, it can be eaten regularly without any problems or undesirable side effects. It is likely already present in very small amounts in our foods. My discovery is that there are great health improvements to be achieved by marked increasing our consumption of it.

Furthermore, I believe that glycine should be considered as an essential product for one's health. Thus, its use should be extended as broadly as possible around the world. It is a product that is absolutely necessary to allow one's metabolism to work well, so it must be taken every day, to guarantee metabolic health, and its intake should be maintained without interruption.

The regular intake of glycine as diet supplement must be increased (e.g., 20-50 grams/day) in some cases to beyond the normal dosages recommended herein of about 10 grams/day. This applies specifically to processes of sinew injury repair, broken bone repair, or in acute cases of all the problems listed in FIG. 1. Furthermore, some of my additional research suggests that it may be advisable in such situations to complement my daily recommended glycine intakes with the consumption of vitamin C (1 g/day or more).

It was earlier mentioned that glucosamine-based drugs are now being orally administered for the treatment of arthrosis and that the basis for this treatment is the hypothesis that arthrosis is due to a failure in proteoglycan biosynthesis, due to a lack of its precursors. However, I have been unable to find any theoretical or empirical evidence supporting this hypothesis.

Rather than increase the synthesis of proteoglycans, my treatments disclosed herein are based on promoting the synthesis of collagen because:
(a) Glycine (H₂N-CH₂-COOH) is a non-essential amino acid that is unique for many reasons, including the fact that one's body has a greater demand for it for biosynthesis purposes than for any other amino acid, and because one's cells are relatively poor in making it in comparison to its broad metabolic needs within our bodies, see FIG. 2.
(b) The main metabolic use of glycine is for collagen biosynthesis; it being a major component of a broad number of mechanical structures in the body.

How such a wide range of health problems can be so significantly impacted by my specified daily dosages of glycine can be understood, in part, by further considering the role of glycine in the body.

Glycine is the smallest amino acid; which, although apparently trivial by its very simple structure, H₂N-CH₂-COOH, has two special properties that make it vital in life chemistry, both for structural purposes, and as a chemical reagent.

Chemical Synthesis: Glycine is a common product in the chemical industry, as raw material for many synthesis processes. The reason of this utility is that it is really a one-carbon-unit (C₁) with two reactive groups (-COOH and -NH₂), which confers on it a high chemical reagent potential for chemical building process. Cell metabolism uses glycine to build complex molecules, such as the heme group and the purine bases, as well as many other simpler ones, such as creatine. Many important metabolic processes depend on molecules made with glycine. Table 2 lists some of the different roles of glycine in the development of different bodily materials and the structures made from these materials, along with the physiological processes and possible health problems associated with these structures.

**Table 2. Different roles of glycine in metabolism, as component of protein structure, and as raw material for the biosysnthesis of other structures and their physiological processes and potential associated health problems.**

| **Material** | **Structure** | **Physiological process** | **Disease/Health Problem** |
|---|---|---|---|
| Collagen | Cartilage | Cartilage renew | Arthrosis, arthritis, osteoarthritis |
| Collagen | Cartilage | Cartilage renew | Discal Hernia |
| Collagen | Cartilage | Cartilage renew | Physical injuries |
| Collagen | Bone | Bone renew | Lose of bone mass |
| Collagen | Bone | Bone renew | Osteoporosis |
| Collagen | Bone | Bone renew | Paget disease |
| Collagen | Bone | Repair of bone injuries | Physical injuries (broken bones) |
| Collagen | Connective tissue, general | Mechanical support | Shape of the body, firm breasts, etc. |
| Collagen | Connective tissue, general | Mechanical support | Fibromyalgia |
| Collagen | Connective tissue, general | Mechanical barriers | Cancer |
| Collagen | Connective tissue, general | Mechanical barriers | Infective diseases |
| Collagen | Connective tissue, sinew | Sinew repair | Sport injuries |
| Collagen | Connective tissue, smooth muscle | Mechanical support | Muscular hernia |
| Collagen | Connective tissue in skin | Mechanical support of skin | Skin lines and wrinkles |
| Collagen | Connective tissue in skin | Mechanical support of skin | Psoriasis |
| Collagen | Connective tissue in nail bed | Mechanical support of nail | Nail weakness |
| Collagen | Connective tissue in hair bed | Mechanical support of hair | Hair loss |
| Collagen | Connective tissue in blood vessels | Mechanical support | Hemorrhage, aneurism |
| Collagen | Connective tissue in eyes | Mechanical support | Blindness, Presbyopia |
| Collagen | Connective tissue in lungs | Mechanical support | Asthma, Emphysema |
| Collagen | Connective tissue in ears | Mechanical support | Hearing problems |
| Collagen | Connective tissue in ears | Mechanical support | Multiple sclerosis |
| Elastin | Yellow connective tissue | Elasticity in junctions | Poor elasticity in junctions |
| Elastin | Yellow connective tissue | Elasticity in the skin | Poor elasticity in skin, wrinkles |
| Heme group | Hemoglobin | Oxygen transport in blood | Anemia |
| Heme group | Myoglobin | Oxygen transport in muscle | Muscle diseases, fatigue |
| Heme group | Myoglobin | Oxygen transport in heart cells | Heart failure |
| Heme group | Cytochromes | Cellular respiration | Red muscle diseases |
| Heme group | Cytochromes | Cellular respiration | Heart failure |
| Heme group | Cytochromes | Cellular respiration | Diseases in other tissues |
| Heme group | Cytochrome P-450 | Liver detoxification system | Drug toxicity |
| Glutathione | Glutathione | Reduction of methemoglobin | Anemia |
| Glutathione | Glutathione | Reduction of metmyoglobin | Muscular diseases |
| Glutathione | Glutathione | Reduction of membrane parts | Anemia and other diseases |
| Glutathione | Glutathione | General antioxidant processes | General oxidative stress |
| Purine bases | Nucleic acids | Nucleic acids synthesis | Anemia |
| Purine bases | Nucleic acids | Nucleic acids synthesis | Tissue regeneration |
| Purine bases | Nucleic acids | Nucleic acids synthesis | Birth defects |
| Bile salts | Bile salt excretion | Fat digestion | Problems in fat digestion |
| Bile salts | Bile salt excretion | Cholesterol excretion | Cholesterol excess |
| Creatine | Muscle structure | Muscle energy traffic | Muscular dystrophies |
| Threonine | Threonine (essential amino acid) | Acetaldehyde removing | Alcoholism, Alcohol intoxication |

(i) The heme group, central component of hemoglobin, myoglobin, cytochromes and some other enzymes (see FIG. 3).
(ii) Purines (nitrogen bases for nucleic acids DNA and RNA), as well as basic material for nucleotides that are component of coenzymes [NAD(P)⁺, Coenzyme A, FAD, ATP, etc].
(iii) Glutathione, a tripeptide that works in metabolism as a general reduction (antioxidant) reagent which intervenes in many reactions and processes maintaining membrane groups, the iron of globins, and also in amino acid transport, and many more processes, probably many of them still unknown.
(iv) Bile salts. Glycocholate, a derivative of cholesterol, which is excreted from the liver to the small intestine where participates in lipid digestion (see FIG. 4).

Protein structure: The role of glycine as an amino acid residue in protein structure is critical, as its small size-the lack of a carbon R-group-allows the protein to have a high capacity for folding, so glycine is like a wild card in occupying difficult sites in protein structure, thereby allowing the structural folding of the protein to be determined by the rest of the amino acids. This property is particularly important in collagen, as it permits the characteristic strong structure of this protein, mainly based on the special folding imposed by proline residues. Elastin also needs glycine to have its elastic properties.

Glycine is theoretically categorized as a non-essential amino acid, because it can be synthesized in human metabolism. Recognizing its simple structure, as given above, one might think that its synthesis should be easy - but it is not. In fact, glycine biosynthesis is difficult, not really because of chemical problems (its synthesis is achieved in a few steps), but because of problems derived from its various metabolic pathways; see FIG. 2.

It starts from 3-phosphoglycerate, which is taken from glycolysis (see step 1 in FIG. 2). Glycolysis is the main route of energy carbohydrate metabolism, and a very large central metabolic pathway, so in principle there are no problems to take all necessary material from there. 3-P-Glycerate is converted into the amino acid serine in three steps (see step 2); these reactions are not difficult, and so there is, in principle, no reason to have a special requirement of serine in the diet, it being a clear non-essential amino acid.

The problem comes next: serine is converted into glycine in just one step (see step 3), which is the elimination of a C₁ unit as a carbacation hydroxymethyl group [⁺CH₂-OH]. This reaction is not especially difficult if there is a coenzyme to transport the C₁ group (see step 4). This coenzyme is tetrahydrofolate (THF), which is a derivative of folic acid, a B-complex vitamin. However, step 3-4 has two problems: (a) THF is at a very low concentration molecule in metabolism (about 100 times less than other B-coenzymes); this means that there is not much of it available, and (b) when serine is converted to glycine (step 3), THF is loaded with the C₁ group (step 4), which must be released (step 5) in order to recover THF so as to catalyze the reaction again.

As FIG. 2 shows, this unloading involves some specific metabolic reactions leading to biosynthesis of purines (step 6), thymine (step 7), or to transfer the methyl group to adenosyl-methionine (step 8), which is, in turn, a coenzyme for most of methyl transfer reactions (step 9). This transfer must be fast in order for THF to be ready again for reaction or step 4, as THF concentration is very low.

The key enzyme in glycine biosynthesis (step 3-4) is glycine hydroxymetiltransferase (EC 2.1.2.1), in whose reaction one molecule of serine is converted into one of glycine plus one C₁ unit which is taken by THF (ser + THF → gly + THF-C₁). Thus, it is not a bifurcation point which could alternatively produce glycine or THF-C₁ with the possibility to deviate the flux toward glycine, but just one reaction with fixed stoichiometry.

In addition to this constraint, there is not a bypass which could allow a rearrangement of the flux toward glycine, as it occurs, e.g., in glycolysis with the triose-phosphate isomerase reaction after the breakdown of the hexose by aldolase. FIG. 3 shows that a bypass exists; it is the glycine cleavage system (EC 1.4.4.2/ 2.1.2.10, (step 13), which catalyzes gly + THF + NAD⁺ → THF-C₁ + CO₂ + NH₃ + NADH; however, the thermodynamic features of this reaction (because of CO₂ release) means that it cannot account for glycine synthesis, but for releasing of a second C₁ unit from glycine degradation, as it can only work in the direction from glycine toward C₁, not in the opposite direction.

An alternative pathway which might produce glycine in metabolism is the reaction of threonine degradation. The enzyme threonine aldolase (EC 4.1.2.5) catalyzes the reaction: threonine → glycine + acetaldehyde. However, the importance of this reaction as a source of glycine is minimal because threonine is an essential amino acid whose intake from the diet is very close to its needs; there is, thus, not enough threonine available to account for both threonine and glycine needs.

Thus, it is clear that glycine biosynthesis depends stoichiometrically on the whole set of reactions dependent on THF-C₁ (Reactions 5-9 in FIG. 3; since each of them is involved in a different physiological process, independent of the glycine needs, the coupling of glycine synthesis with any of them is not obvious. A strong requirement of glycine (as it is necessary for the biosynthesis of many products, Reactions 10-13) has to force an equivalently high biosynthesis rate of these other compounds (5-9) which then would led to a number of undesirable deviations of metabolic fluxes increasing other parts of the metabolism not necessary to occur in such a quantity, and thus creating a number of metabolic problems.

Thus, glycine, although having a very simple chemical structure, has a very difficult biosynthetic pathway. Since it is the most used amino acid in metabolism, and since it is difficult to produce freely in high amounts, the result is that glycine is needed in our diets.

Human beings and many domesticated animals need a daily, heretofore unrecognized, high amount of glycine in their diets. Thus, these facts lead us to the conclusion that many adverse health conditions in which the affected parts of the body are its connective tissues, cartilage and bones, including some degenerative diseases, should be really considered as conditions brought about by dietary deficiencies.

To help one to better understand how such treatment benefits can be achieved as a result of my recommended glycine treatment programs, consider the following:

### Degenerative Diseases:

A theoretical basis for why my results should be expected is based on the following hypotheses:
(a) In every degenerative disease some specific metabolic problem exists, i.e., there is some part of the metabolism which is not working correctly, or some part of the cellular chemical machinery (metabolism) has not enough capacity to fulfill the cellular requirements for some specific process. I call these special locations in the metabolic map "weak points of metabolism". There are, in principle two kinds of weak points: those caused by a deficiency (empty points) and those caused by an excess (crowd points)
(b) It is possible to relate these weak points of metabolism with the composition of the diet, which supplies the material for metabolic work. In principle, the empty points are consequence of a deficiency of some specific nutrients, while the crowd ones are consequence of excess of others. The action I am recommending herein is to repair the weak points by dietary changes, by correcting the composition of the nutrients according to the specific necessities of metabolism.
(c) Enzymes work in cellular metabolism with a very low mean saturation degree. For instance, data obtained for glycolysis shows a mean saturation percentage of around 20% (i.e., only two of each ten enzyme molecules are working). This feature gives us a broad opportunity to correct many problems in metabolic pathways, even problems with a specific genetic origin.

In a degenerative disease, as a consequence of the deficiency of the regular enzyme synthesis, the amount of enzyme is reduced. For example, let us consider an enzyme whose normal amount in a healthy individual is 10 enzyme molecules, from which only 2 work (20%). An individual affected with a degenerative disease would have a fewer amount of this enzyme (e.g., half the normal quantity, 5). Thus, since it is not possible to increase the amount of the enzyme, 2 working molecules can only be achieved by increasing the mean saturation percentage, and this can be done by means of increasing the amount of the metabolite intermediates (through the composition of one's diet).

The general conclusion of this reasoning is a strategy for the treatment of degenerative diseases, including those with a genetic origin: treat them as if they are "deficiency diseases" rather than degenerative diseases.

Anemia: To explain these treatment benefits, it is my observation that there is a closer relationship between anemia and glycine than has heretofore been recognized. FIG. 3 shows the structures of heme (the prosthetic group of hemoglobin) and porphobilinogen (the basic intermediate in heme building). Two glycines are used as raw materials to build each porphobilinogen molecule, and four porphobilinogen molecules are used to build one heme group. Thus, in FIG. 3 can be seen that eight carbon atoms and the four nitrogens in the heme structure (marked in bold in the scheme) come from glycine. It can also be seen that eight molecules of glycine are spent to build one heme. Hemoglobin is a very abundant protein in the body representing 90% of the dried weight of the red blood cells (around 2×10¹² cells in the body of an average adult). Moreover, hemoglobin turnover is short (120 days) and there is no recycling of material, as the heme group is destroyed to bile pigments. Blood building is, thus, a high expenditure of glycine, and so its deficiency can be a very probable cause of anemia.

Fetus Development: During pregnancy, nucleic acid synthesis is highly increased in the development of the fetus, involving an obvious demand of purine and pyrimidine synthesis, to which a high supply of Ci units and the corresponding folic acid availability to transfer them is required (see Reactions 4-7 in FIG. 2) in order to prevent birth defects, particularly neural tube defects. The conversion of homocysteine to methionine (Reaction 6 in FIG. 2) has been proposed as a complementary hypothesis to explain this requirement. The general conclusion is that pregnant women must increase the daily intake of folic acid from the regular dose previously stated of (50 *µ*g) to 400-800 *µ*g, and even to 4-5 mg.

Considering the difficulty for glycine synthesis in metabolism discussed above, due to the special stoichiometric features of Reaction 3, it is clear that folic acid favors this reaction. Thus, these interactions strongly suggest that the special needs of folic acid in pregnancy really mean a special need for glycine - which must obviously be higher than in the regular life so as to build the entire mechanical structure of the fetus.

All this reasoning can be summarized saying that a strong mechanical structure of the body is necessary to avoid birth defects. Thus, I contend that during pregnancy there is a strong need of glycine, which should be supplied as a diet supplement in high amounts.

Cholesterol Excesses: Bile salts are cholesterol derivatives (see FIG. 6) that are excreted by the liver to the small intestine where they are used as emulsifying agents for lipid digestion. About 20-30 g of bile salts is excreted every day and most of this material returns to the liver by enterohepatic circulation, so only 400 mg (1.5-2.0% of the excreted material) are fully eliminated every day. The rate of cholesterol synthesis is about 800 mg/day in a 80 kg person; one half is excreted as bile salts, and the rest is converted in minor compounds, such as hormones (50 mg), or used for other purposes, and some of it can be accumulated in the body every day.

Synthesis of the bile salts is the regular way for cholesterol excretion, as they are soluble compounds and easy to eliminate. Some specialists have suggested that a way to reduce plasma cholesterol would be if one were capable of reducing the resorption of bile salts in the enterohepatic circulation.

Noting that glycine is around 6% of the mass of bile salts (see FIG. 4), I have observed that if they were not resorbed, the daily loss of glycine through that pathway would be 1.0-1.5 g, which is approximately one's regular intake; thus, leaving no glycine for other uses. Thus, it appears to me that the main reason why we have heretofore had to have the resorption of bile salts (i.e., to avoid their depletion in the body) could henceforth be made unnecessary if one were to supplement their diet with glycine - resorption of bile salts would no longer be necessary and consequent blood cholesterol levels should decrease, i.e., the glycine diet supplement reduces one's plasma cholesterol by means of increasing the synthesis, and the consequent excretion of bile salts, reducing the need for their resorption.

Table 2 shows my broad (although probably not complete) view of the metabolic roles of glycine as a precursor for the development of different cell and tissue structures, and the problems that can occur if there are not enough amounts of each material for adequate structure development. I have not yet been able to experimentally demonstrate all of these relationships, but I believe that a form theoretical basis exists for my suggestion of these relationships.

These relationships suggest other health problems or situations that possibly could be treated with appropriate daily dosages of glycine, including:
Heart Conditions: Atherosclerosis ("hardening of the arteries") is a well-known progressive heart disease in which deposits of lipids and cholesterol accumulate in the inner arterial wall. Atherosclerosis is strongly correlated with one's level of plasma cholesterol, but its onset appears to be triggered when the endothelial layer of the vessel is damaged. This injury then provokes an immune reaction, which increases the chances that the cholesterol and lipid deposits will accumulate. I believe that a weakness of the connective tissue, due to an insufficient synthesis of collagen, can provoke such vessel damage. Thus, it may be that the origin of a number of cardiovascular problems is a weakness in the mechanical structure of the heart. For example, a weakness in a heart vessel can produce aneurysm (i.e., a permanent arterial enlargement usually caused by weakening of the vessel wall). Such conditions may be treatable with daily glycine dosages.

Collagen and elastin are important components of the heart, since it has an abundant mass of connective tissue that is rich in collagen and elastin, with this tissue both supporting the organ and its mechanical function. There are many heart diseases that are associated with some kind of connective tissue problems that may be due, in part, to collagen and elastin deficiencies. I suggest that insufficient collagen biosynthesis is often due to glycine deficiency, which can be treated by adding glycine to one's diet.

Hemorrhages: The speculation that this health problem can potentially be treated with daily glycine dosages follows directly from my earlier discussion of the impact of glycine consumption on the heart's blood vessels.

Disc Hernias: The speculation that this health problem can potentially be treated with daily glycine dosages follows directly from my earlier discussions related to the importance of increasing the synthesis of collagen in the body.

Aid To The Development of One's Skeletal System: My research suggests that children would benefit from a daily consumption of glycine. Appropriate dosages would appear to be: for children seven and under: 0.3 - 0.7, with a preference for 0.5, g/Kg of body weight, for children 8-18: same dosage as for adults.

Aid To Postoperative Recovery: The speculation that this health condition (i.e., the need to promote healing from one's surgical wounds) can potentially be treated with daily glycine dosages follows directly from my earlier discussion of the impact of glycine consumption on the development and health of the body's mechanical components.

Minimize Skin Damage Due To Sun Exposure: The speculation that this potential health situation can potentially be minimized or treated by adding glycine, in appropriate therapeutic amounts, to assorted sun tanning products follows directly from my earlier discussion of the impact of glycine consumption on the development and health of the body's mechanical components.

Aid In Helping One's Skin Adapt To A New Body Size After Significant Weight Reductions: The speculation that this situation can be addressed by adding glycine, in appropriate therapeutic amounts, to assorted weight loss products or by having glycine be ingested as a dietary supplement follows directly from my earlier discussion of the impact of glycine consumption on the development and health of the body's mechanical components.

Aid In Helping A Woman's Skin Recover From A Pregnancy: The speculation that this situation can be addressed by adding glycine, in appropriate therapeutic amounts, to assorted lotions and creams, etc. or by having glycine be ingested as a dietary supplement follows directly from my earlier discussion of the impact of glycine consumption on the development and health of the body's mechanical components.

Aid In Helping A Man Address Erectile Dysfunction and Impotencv Problems: The speculation that this situation can be addressed by adding glycine, in appropriate therapeutic amounts, to assorted lotions and creams, etc. or by having glycine be ingested as a dietary supplement follows directly from my earlier discussion of the impact of glycine consumption on the development and health of the body's mechanical components.

Means For Increasing The Probability of Success In In-Vitro Fertilization Procedures: The speculation that this situation can be addressed by oral consumptions of glycine follows directly from my earlier discussion of the impact of glycine consumption on the development and health of the body's mechanical components and an awareness of the importance of the health of the uterus in such procedures.

Aid To Drug Detoxification: The speculation that this situation can be addressed by oral consumptions of glycine follows directly from my earlier discussion of the metabolic pathways involved in glycine biosynthesis, see FIG. 3, and an awareness of the importance of promoting cytochrome P450 activity as part of such drug detoxification processes.

Aid To Promote The Growth and Health of Cultivated Or Fish-Farm Fish: The fish farm industry continues to grow as its fish products become more widely accepted in the marketplace. The speculation that this industry can be aided by adding glycine to the fish foods uses by these producers follows directly from my earlier discussion of the impact of glycine consumption on the development of the bones, etc.

Moving now from these speculations and potential applications for glycine, it is instructive to try to develop some theoretical basis or justifications for my recommendations regarding a human's optimal daily intake of glycine. These are given below:
Collagen is known to be one of the body's slow turnover proteins. Its biosynthesis rate is estimated between 2 and 5 grams per day in young adults, and approximately half of that in older adults. For young adults with a zero growth rate, we assume that the rate of collagen degradation is the same as that its biosynthesis.

If glycine is about one-third of the amino-acid residues of collagen, and about one-fifth of its mass, a daily collagen turnover of 2-5 g of collagen implies a daily consumption of between 300 and 1,000 mg of glycine for collagen biosynthesis. Taking into account the other metabolic ends of glycine (see Table 2), let us speculate that about 1.0-2.5 grams are necessary daily. Since no chemical reaction uses its available raw materials at maximum efficiency, I further speculate that one's body needs four times or more of these amounts.

While considering glycine's optimal daily intake rate, it may be informative to compare this recommended rate with an estimate of how much glycine an average person might presently be consuming. Our primary natural source of glycine intake is via our meat consumption which contains relatively large amounts of collagen (the only abundant protein with high glycine content). The scarcity of collagen in our regular diet is probably the origin of our glycine deficiencies, since most people's eating habits often involve avoiding high collagen items: bones, fish-bones, cartilages, skin, meat's connective tissues in the meat. Additionally, it should be noted that it is not clear how much of the collagen we eat is digested to polypeptides and amino acids, and so, assimilated as a nutrient.

One's normal intake of glycine is estimated, probably on the high side, as follows: glycine is about 5% of the mass of many proteins (note: ∼ 20% of collagen's mass). Assume one's regular intake of protein meals (meat, jam, fish, eggs, etc.) is about 150 g per day. Assume that water makes up most of these meals and only about one-fifth are proteins. This implies a regular intake of 30 g of protein per day. Thus, our normal intake of glycine is around 5% of 30 g or 1.5 g.

The dosage I often recommend herein (a supplement of 10 g daily) implies increasing our normal intake by about 7 times. This is roughly equivalent to the increase (i.e., a factor of 10 times) used regularly in biochemical experiments to enhance significantly the activity of enzymes.

It should be noted that the calculations given above for glycine needs are based on data which indicates that collagen is a slow turnover protein, with an estimated lifetime of about four years. Thus, one might expect the time necessary for the repair of an injured joint to be years, but we know that bone breaks heal in a matter of weeks. Thus, collagen turnover can apparently be much faster in certain situations. I suspect that my recommended glycine treatments may be creating such situations, as older participants in the studies reported herein showed significant decreases in the adverse effects of arthrosis on their joints after durations of glycine treatments of only 2 -3 weeks. Collagen biosynthesis and the rebuilding of collagen apparently is much faster under metabolic conditions of glycine abundance brought about by, among many such means, the ingestion of glycine as a dietary supplement.

Do humans really have such a significant dietary deficiency and, if so, what is the origin of this problem? How could our metabolism be designed in such a way that a substance (i.e., glycine) so necessary is so scarce and difficult to obtain? I believe that an explanation is to be found in that fact that humans were in their origins essentially carnivorous beings. They subsequently cultivated and developed a whole host of foods based mainly on carbohydrates: wheat, corn, rice, and other cereals, and their derivatives, such as bread and pasta, and other agricultural food rich in carbohydrates, such as pulses (beans, chickpeas, lentils, etc), and potatoes. I believe that most of the health problems listed in FIG. 4 are probably the consequence of this change in our eating habits.

The foregoing is considered as illustrative only of the principles of the invention. Further, since numerous modifications and changes will readily occur to those skilled in the art, and because of the wide extent of the teachings disclosed herein, the foregoing disclosure should not be considered to limit the invention to the exact methods shown and described herein. Accordingly, all suitable modifications and equivalents of the present disclosure may be resorted to and still considered to fall within the scope of the invention as hereinafter set forth in the claims.

## Claims

1. Composition consisting of glycine for use in the treatment of a health problem of the skin in a human in the need of promoting the synthesis of collagen, in which said glycine is orally administered at a consumption rate in the range of 0.1 to 0.2 g/Kg of body weight per day.

2. A composition for use according to claim 1, in which said problem is the healing of a surgical wound.

3. A composition consisting of glycine for use according to claim 1, **characterized in that** the dosage of glycine is greater than 5 grams per day.

4. A composition consisting of glycine for use according to claim 3, **characterized in that** the dosage of glycine is 10 grams per day.

5. A composition consisting of glycine for use according to claim 3, **characterized in that** the dosage of glycine is greater than 10 grams per day.

6. Use of a composition consisting of glycine in the non-therapeutical pigmentation of the skin, treatment of wrinkles, sagging and scars in a human in the need of promoting the synthesis of collagen, in which said glycine is orally administered at a consumption rate in the range of 0.1 to 0.2 g/Kg of body weight per day.

7. A cream able to be applied on the skin, comprising glycine.

8. The cream of claim 7, consisting in glycine as active agent.

9. The cream according to claim 7 or 8, in which said glycine is present in a rate of between 500 and 1700 mg/ 100 g of cream.

10. The cream of any one of claims 7 to 9, for use in the treatment of psoriasis.

11. Use of the cream of any one of claims 7 to 9, in the non-therapeutical treatment and prevention of skin lines of a human.
